# EUROPEAN PATENT APPLICATION

(11) **EP 1 122 239 A2**
(43) Date of publication of application: **08.08.2001**
(21) Application number: 00308751.7
(22) Date of filing: 04.10.2000
(51) Int. Cl.: C07C 303/38, C07C 311/48

(54) **Process for producing sulfonylimide compound**

(30) Priority: 31.01.2000 JP 2000021578
(71) Applicant: Morita Chemical Industries Co., Ltd., Osaka-shi, Osaka 531-0072 (JP)
(72) Inventor: Yonezawa, Tetsuo, c/o Morita Chem. Ind. Co., Ltd., Osaka-shi, Osaka 532-0002 (JP); Sakamoto, Yoshitaka, Morita Chem. Ind. Co., Ltd., Osaka-shi, Osaka 532-0002 (JP)
(74) Representative: Brasnett, Adrian Hugh

(57) **Abstract**

The present invention pertains to a process for producing sulfonylimide compounds (represented by the formula (I) MN(SO₂R_{f}¹) (SO₂R_{f}²)) industrially easily at a low cost in an efficient manner, wherein the process comprises reacting a sulfonyl fluoride represented by the formula (II) R_{f}SO₂F with ammonia anhydride or an ammonium salt in the presence of a fluorine compound represented by the formula (III) MF. The above M represents any one of Li, Na, K and Cs among alkali metals of group Ia in the periodic table. The above R_{f}¹ and R_{f}², which may be the same of different, respectively represent any one of a straight chain or branched compound of a fluoroalkyl, perfluoroalkyl, fluoroallyl or fluoroalkenyl group having 1 to 12 carbon atoms, and R_{f} in the above formula (II) represents the same group as R_{f}¹ or R_{f}² in the formula (I).

## Description

### TECHNICAL FIELD OF THE INVENTION

The present invention relates to a process for producing a sulfonylimide compound represented by the formula (I):

MN (SO₂R_{f}¹) (SO₂R_{f}²).

### BACKGROUND

Sulfonylimide compounds are safe as a solute of a battery electrolyte and battery electrolyte using the sulfonylimide compound as the solute has a high energy density and exhibits high conductivity. Hence the sulfonylimide compounds are regarded as a promising solute of a battery electrolyte.

Also, the sulfonylimide compounds are useful as a Lewis acid catalyst and an ionic conduction agent.

The sulfonylimide compounds represented by the formula (I) MN (SO₂R_{f}¹) (SO₂R_{f}²) may be synthesized by the process proposed by D.D. Desmarteau et al. in INORGANIC CHEMISTRY, VOL. 23, No. 23, pp. 3720-3723 (1984).

This synthetic method is, as shown by the following formula, a method in which trifluoromethanesulfonyl fluoride is reacted with ammonia anhydride, the resulting product is treated using hydrochloric acid to produce trifluoromethanesulfonylamide, which is then reacted with sodium methylate and then with hexamethyldisilazane and the resulting product is reacted with trifluoromethanesulfonyl fluoride to obtain an imide sodium salt.

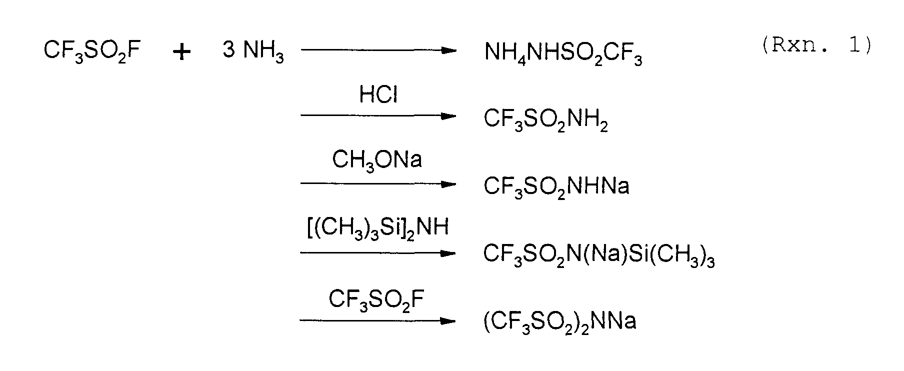

However, this process involves multi-reaction steps and is hence made longer. Also, expensive hexamethyldisilazane must be used to obtain an intermediate and the yield is as low as about 50%.

Also, in Japanese Published Searched Patent Publication No. Hei3-501860, a method is disclosed in which a silazane metal compound is reacted with a perfluorosulfonyl halide compound to obtain an imide compound. In Japanese Published Searched Patent Publication No. Hei4-501118, a method is disclosed in which an ionic nitride is reacted with a halogenated sulfonic acid to obtain an imide compound.

However, the silazane metal compound and the ionic nitride are all expensive and hence the above methods are not said to be an economic production method.

Also, in the publication of JP-A-8-81436, a method is disclosed in which ammonium anhydride or a sulfonylamide and a sulfonyl fluoride are reacted with a tertiary amine or a heterocyclic amine and the reaction product is further reacted with, for instance, a hydroxide containing an alkali metal and an alkaline earth metal to produce imide salts.

In this method, because the product in the first stage is generated as an amine salt, it must be further reacted with an inorganic salt. Also, a tertiary amine or a heterocyclic amine is used in the reaction, posing problems concerning work environment caused by the odour and disposal of the amine. Moreover, because the ammonia anhydride is always used, an autoclave as the reactor and a low temperature cooling unit are required. This method is therefore unsuitable for mass-production.

As outlined above, the prior art involves a long reaction step and uses expensive raw materials and it is hence hard to say that these methods in prior art are industrially acceptable methods.

Also, in JP-A-8-81436, ammonia anhydride, a perfluoroalkanesulfonyl fluoride and a tertiary amine are reacted with each other. To obtain an imide salt, at least two steps are required and in the reaction, a tertiary amine or a heterocyclic amine is used, affording possibility for pollution of work environment caused by the odour and the like. Further, the product must be reacted with an alkali metal or the like in an aqueous solution in the second step. At this time, it is necessary to dispose the amine which is freed and distilled together with water, causing increased production costs.

It is an object of the present invention to solve these various problems and to produce a sulfonylimide compound industrially easily at a low cost in an efficient manner.

### SUMMARY OF THE INVENTION

The inventors of the present invention have found that a sulfonylimide compound (represented by the formula (I) MN(SO₂R_{f}¹) (SO₂R_{f}²)) which is free from the foregoing problems can be produced industrially easily at a low cost in an efficient manner.

Accordingly, one aspect of the present invention pertains to a process for producing a sulfonylamide compound comprising reacting a sulfonyl fluoride represented by the formula (II) R_{f}SO₂F with ammonia anhydride or an ammonium salt in the presence of a fluorine compound represented by the formula (III) MF.

In the aforementioned formula (I), R_{f}¹ and R_{f}², which may be the same of different, respectively represent any one of a straight chain or branched compound of a fluoroalkyl, perfluoroalkyl, fluoroallyl or fluoroalkenyl group having 1 to 12 carbon atoms (the same hereafter).

M in the aforementioned formulae (I) and (III) represents any one of Li, Na, K and Cs among alkali metals of group Ia in the periodic table (already explained, the same as follows). Also, R_{f} in the aforementioned formula (II) represents the same group as R_{f}¹ or R_{f}² in the formula (I) (the same as follows).

Examples of sulfonylimides include, but are not limited to, the following: LiN (SO₂CF₃)₂, NaN (SO₂CF₃)₂, KN(SO₂CF₃)₂, CsN(SO₂CF₃)₂, LiN(SO₂C₄F₉)₂, NaN(SO₂C₄F₉)₂, KN(SO₂C₄F₉)₂, CsN(SO₂C₄F₉)₂, LiN(SO₂CF₃) (SO₂C₄F₉), NaN(SO₂CF₃) (SO₂C₄F₉), KN(SO₂CF₃) (SO₂C₄F₉), and CsN(SO₂CF₃) (SO₂C₄F₉).

Another aspect of the present invention pertains to a process for producing a sulfonylamide compound represented by the formula (I) MN(SO₂R_{f}¹) (SO₂R_{f}²) by only one stage reaction under mild conditions comprising reacting a sulfonylamide represented by the formula (IV) R_{f}SO₂NH₂, a sulfonyl fluoride represented by the formula (II) R_{f}SO₂F, and fluorine compound represented by the formula (III) MF with each other.

There exist Li, Na, K, Rb, Cs and Fr as the alkali metals of Ia group in the periodic table. Among these metals, especially any one of Li, Na, K and Cs may be selected and the corresponding fluorine compound used accordingly. Therefore, in the case of these metals, the fluorine compounds are LiF, NaF, KF (as KF, any one of calcine-dried KF (cd KF) produced by a smoking method and spraydried KF (sd KF) by a spray drying method may be used) and CsF.

Among the alkali metals of Ia group in the periodic table, Li, Na, K and Cs are preferred. Among these preferable metals, particularly a fluorine compound of K, namely KF is preferable to produce a sulfonylimide compound industrially easily at a low cost in an efficient manner.

In the present invention, on the other hand, the sulfonylimide compound may be produced by using an ammonium salt. As the ammonium salt in this case, it is desirable to use ammonium fluoride or acidic ammonium fluoride. The use of either one of these compounds has the advantage that a specific reactor (pressure container) is not required.

### DETAILED DESCRIPTION OF THE PREFERRED EMBODIMENTS

The embodiments of the invention will be hereinafter explained in detail.

The object compound, which has previously been produced in a multi-step process, can be produced in one step by introducing a fluorine compound represented by the formula (III), a sulfonyl fluoride represented by the formula (II) R_{f}SO₂F, and ammonia anhydride or an ammonium salt into an inert solvent and reacting the mixture as shown by the following reactions 2, 3, 4 and 5.

This is due to the basicity of the fluorine compound represented by the formula (III) MF.
(1) In the case where R_{f}¹ and R_{f}² in the formula (I) MN(SO₂R_{f}¹) (SO₂R_{f}²) are equal to each other: 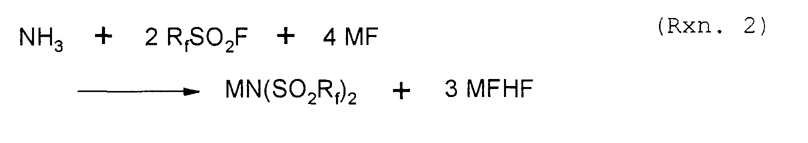
   1 mol of ammonia anhydride, 2 mol of a sulfonyl fluoride represented by the formula (II) R_{f}SO₂F and 4 mol of a fluorine compound represented by the formula (III) MF are introduced into a reactor and the mixture is reacted in a solvent.
   After completion of the reaction, 3 mol of the by-product acidic fluoride MFHF is removed by filtration and the filtrate is concentrated. The sulfonylimide compound represented by the formula (I) MN(SO₂R_{f})₂ can be thereby produced.
(2) In the case where R_{f}¹ and R_{f}² in the formula (I) MN (SO₂R_{f}¹) (SO₂R_{f}²) are different from each other:
   A sulfonylamide containing the R_{f}¹ group which is produced through a known process as shown in the following is reacted with a sulfonyl fluoride having a desired R_{f}² group. The sulfonylimide compound represented by the formula (I) MN(SO₂R_{f}¹) (SO₂R_{f}²) can be thereby produced. 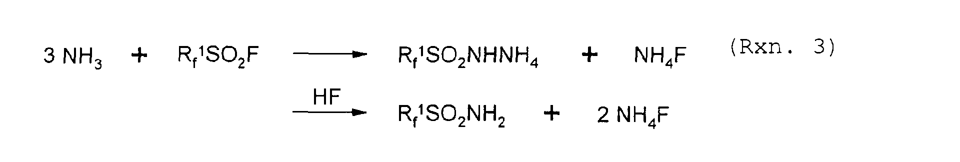 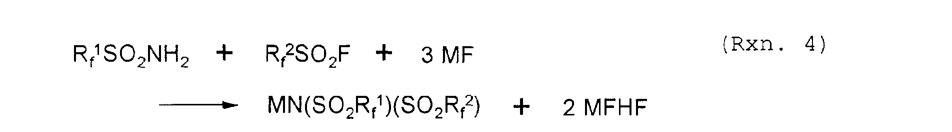
(3) In the case of using an ammonium salt: 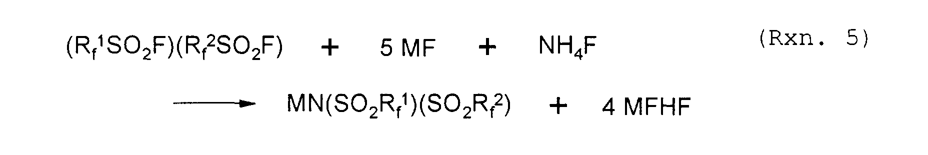 wherein R_{f}¹ and R_{f}² are the same or different.
   1 mol of an ammonium salt, 2 mol of a sulfonyl fluoride represented by the formula (II) R_{f}SO₂F and 5 mol of a fluorine compound represented by the formula (III) MF are introduced into a reactor and the mixture is reacted in a solvent.

After completion of the reaction, 4 mol of the by-product acidic fluoride MFHF is removed by filtration and the filtrate is concentrated. The sulfonylimide compound represented by the formula (I) MN(SO₂R_{f}¹) (SO₂R_{f}²) can be thereby produced.

Although MF reacts when its amount is both more and less than the number of mols described above, the reaction efficiency is made lower.

These reactions can be run in a temperature range between about -30°C and 200°C. At a temperature less than the above range, the reaction rate is very low whereas at a temperature exceeding the above range, decomposition of the compounds, solvent and product to be used arises.
A more preferable temperature range is between 0°C and 100°C.

As to the solvent which can be used, any solvent may be used without particular limitations as far as it is inert to the reaction materials. For example, ethers such as diethyl ether and tetrahydrofuran, halogenated hydrocarbons such as dichloromethane and dichloroethane, hydrocarbons such as benzene, heptane and hexane and nitriles such as acetonitrile may be used.

In order to produce various sulfonylimide compounds other than those mentioned above, a sulfonylimide compound obtained by these production methods may be made into an acid by using concentrated sulfuric acid and distilling the acid to thereby synthesize a sulfonylimidic acid [HN(SO₂R_{f}¹) (SO₂R_{f}²)]. This acid may be further reacted with a compound selected from hydroxides, oxides, carbonates and acetates of metals corresponding to this acid.

In this case, fluorine compounds represented by the formula (III) MF to be used in the synthesis of a sulfonylimide compound may be used.

The present invention will be explained in more detail by way of examples, which, of course, are not intended to be limiting of the present invention.

### Example 1

A flask with four necks was charged with 150 ml of acetonitrile, 23.4 g of potassium fluoride and 20 g of trifluoromethanesulfonylamide. The reactor was soaked in a 40°C hot water bath and 25.1 g of trifluoromethanesulfonyl fluoride was introduced with sufficient stirring. The reaction solution was subjected to filtration and the filtrate was concentrated under reduced pressure to obtain potassium bistrifluoromethanesulfonylimide in an amount of 42.7 g. The yield was 99%.

Next, 42.7 g of this potassium bistrifluoromethanesulfonylimide was added in a flask charged with 60 ml of concentrated sulfuric acid and the mixture was dissolved under heat. 34.6 g of bistrifluoromethanesulfonylimidic acid was distilled under reduced pressure by distillation. The yield was 92%.

Then, 34.6 g of the resulting bistrifluoromethanesulfonylimidic acid was dissolved in pure water and reacted with 4.5 g of lithium carbonate. Excess lithium carbonate was removed by filtration and the filtrate was concentrated to obtain 34.6 g of lithium bistrifluoromethanesulfonylimide. The yield was 98%.

### Example 2

An autoclave made of SUS (stainless) was charged with 200 ml of acetonitrile and 68.3 g of potassium fluoride.

The reactor was cooled to -60°C in a dry ice/methanol bath and 5 g of ammonia anhydride was introduced.

In succession, 50.5 g of trifluoromethanesulfonyl fluoride was introduced and the temperature of the mixture was returned to ambient temperature with sufficient stirring. After that, the reactor was soaked in a 40°C hot water bath and the reaction was completed while stirring sufficiently. The reaction solution was subjected to filtration and the filtrate was concentrated under reduced pressure to obtain 88.2 g of potassium bistrifluoromethanesulfonylimide. The yield was 94%.

### Example 3

A flask with four necks was charged with 1 litre of methylene chloride, 10 g of ammonium fluoride and 78.4 g of potassium fluoride. The reactor was soaked in a 40°C hot water bath and 82.1 g of trifluoromethanesulfonyl fluoride was introduced while stirring sufficiently. The reaction solution was subjected to filtration and the filtrate was concentrated under reduced pressure to obtain 81.5 g of potassium bistrifluoromethanesulfonylimide. The yield was 95%.

### Example 4

A flask was charged with 300 ml of DMF (dimethylformamide), 30 g of perfluorobutanesulfonyl fluoride, 15.1 g of trifluoromethanesulfonylamide and 13 g of sodium fluoride and the mixture was heated to 100°C and sufficiently stirred to react. The reaction solution was subjected to filtration and the filtrate was concentrated under reduced pressure to obtain 35.1 g of sodium perfluorobutanesulfonyl-trifluoromethane-sulfonylimide. The yield was 78.0%.

### Example 5

A flask with four necks was charged with 200 ml of acetonitrile, 12 g of perfluorobutanesulfonyl fluoride and 10.2 g of cesium fluoride and the mixture was heated to 50°C and sufficiently stirred to react. The reaction solution was subjected to filtration and the filtrate was concentrated under reduced pressure to obtain 12.5 g of cesium bisperfluorobutanesulfonylimide. The yield was 88.0%.

### Example 6

An autoclave made of SUS (stainless) was charged with 100 ml of methylene chloride, 100 ml of DMF (dimethylformamide) and 30.5 g of lithium fluoride. The reactor was cooled to -60°C in a dry ice/ methanol bath and 5 g of ammonia anhydride was introduced. In succession, 50.5 g of trifluoromethanesulfonyl fluoride was introduced and the temperature of the mixture was returned to ambient temperature with sufficient stirring. After that, the reactor was soaked in a 50°C hot water bath and the reaction was run while stirring sufficiently. The reaction solution was subjected to filtration and the filtrate was concentrated under reduced pressure to obtain 1.7 g of lithium bistrifluoromethanesulfonylimide. The yield was 2.0%.

Although, at the present stage, the amount and percentage yield of the product compound in this case are lower than in the case of other examples, the reaction may be optimized to improve yield.

It is to be noted that the sulfonylimide compounds obtained in the above examples were respectively confirmed by identification using infrared absorption spectroscopy.

When the production process according to the present invention is used, this has such an effect that sulfonylimide compounds useful as lithium battery electrolytes and organic synthetic catalysts can be produced industrially, easily, at a low cost, and in an efficient manner.

When the production process according to present invention is used in particular, this has such an effect that by reacting a sulfonylamide, a sulfonyl fluoride and a fluorine compound with each other, a sulfonylimide compound useful as lithium battery electrolytes and organic synthetic catalysts can be produced under mild conditions in one stage.

Also, the invention according to the present invention has such an effect that even if ammonia anhydride is not always used, a sulfonylimide compound useful as lithium battery electrolytes and organic synthetic catalysts can be produced under mild conditions in one stage and also has the advantage that a specific reactor (pressure container) is not required unlike the case of using ammonia anhydride.

## Claims

1. A process for producing a sulfonylimide compound represented by the formula (I):
MN(SO₂R_{f}¹) (SO₂R_{f}²)
wherein:
M represents any one of Li, Na, K and Cs among alkali metals of group Ia in the periodic table; and,
R_{f}¹ and R_{f}², which may be the same of different, respectively represent any one of a straight chain or branched compound of a fluoroalkyl, perfluoroalkyl, fluoroallyl or fluoroalkenyl group having 1 to 12 carbon atoms;
the process comprising reacting:
(a) a sulfonyl fluoride represented by the formula (II):
R_{f}SO₂F
wherein R_{f} represents the same group as R_{f}¹ or R_{f}² in the formula (I); with
(b) ammonia anhydride or an ammonium salt; and with
(c) a fluorine compound represented by the formula (III):
MF
wherein M represents any one of Li, Na, K and Cs among alkali metals of group Ia in the periodic table.

2. A process according to claim 1, wherein step (b) employs an ammonium salt.

3. A process according to claim 1 or 2, wherein an ammonium fluoride or an acidic ammonium fluoride is used as the ammonium salt.

4. A process for producing a sulfonylimide compound represented by the formula (I):
MN(SO₂R_{f}¹) (SO₂R_{f}²)
wherein:
M represents any one of Li, Na, K and Cs among alkali metals of group Ia in the periodic table;
R_{f}¹ and R_{f}², which may be the same of different, respectively represent any one of a straight chain or branched compound of a fluoroalkyl, perfluoroalkyl, fluoroallyl or fluoroalkenyl group having 1 to 12 carbon atoms;
the process comprising reacting:
(a) a sulfonylamide represented by the formula (IV) :
R_{f}SO₂NH₂
wherein R_{f} represents the same group as R_{f}¹ or R_{f}² in the formula (I); with
(b) a sulfonyl fluoride represented by the formula (II):
R_{f}SO₂F
wherein R_{f} represents the same group as R_{f}¹ or R_{f}² in the formula (I); and with
(c) a fluorine compound represented by the formula (III) :
MF
wherein M represents any one of Li, Na, K and Cs among alkali metals of group Ia in the periodic table.

5. A process according to any one of claims 1 to 4, wherein R_{f} is CF₃ or C₄F₉.

6. A process according to any one of claims 1 to 5, wherein M is Li, Na, K, or Cs.

7. A process according to any one of claims 1 to 5, wherein M is K.

8. A process according to any one of claims 1 to 6, wherein the compound of formula (I) is LiN(SO₂CF₃)₂, NaN(SO₂CF₃)₂, KN(SO₂CF₃)₂, CsN(SO₂CF₃)₂, LiN(SO₂C₄F₉)₂, NaN(SO₂C₄F₉)₂, KN(SO₂C₄F₉)₂, CsN(SO₂C₄F₉)₂, LiN(SO₂CF₃) (SO₂C₄F₉), NaN(SO₂CF₃) (SO₂C₄F₉), KN(SO₂CF₃) (SO₂C₄F₉), or CsN(SO₂CF₃) (SO₂C₄F₉).

9. A process according to any one of claims 1 to 6, wherein the compound of formula (I) is LiN(SO₂CF₃)₂, KN(SO₂CF₃)₂, CsN(SO₂C₄F₉)₂, or NaN(SO₂C₄F₉) (SO₂CF₃).
